# EUROPEAN PATENT APPLICATION

(11) **EP 2 027 876 A1**
(43) Date of publication of application: **25.02.2009**
(21) Application number: 07253833.3
(22) Date of filing: 27.09.2007
(51) Int. Cl.: A61L 9/04, A61L 9/12, A61L 9/22, F24F 3/16

(54) **Air purifier and air purification method**

(30) Priority: 10.08.2007 WO PCT/SG2007/000246
(71) Applicant: Breathe AP Pte Ltd., Singapore 486132 (SG)
(72) Inventor: Chua, Ee Mong c/o Breathe AP Pte Ltd, Singapore 486132 (SG)
(74) Representative: Wilson Gunn

(57) **Abstract**

An air purifier (100) and air purification method which comprises a processing unit (300) coupleable to a container (204). The processing unit receives air from the enclosed environment for processing such as purifying, ionizing and aromatizing. The processing unit comprises en impeller (308) for drawing ambient air into the processing unit, an ionizer (400) for ionizing the ambient air to obtain first processed air and a funnel (312) shaped for drawing liquid from the container and for substantially centrifugally expelling the liquid adjacent to the impeller for forming a liquid curtain (320). The impeller is for directing the first processed air trough the liquid curtain to obtain second processed air. The second processed air contains liquid molecules absorbed from the liquid curtain. The second processed air is subsequently exhausted into the atmosphere. The funnel is rotatable, conically shaped, whereby the narrow end is in contact with the liquid to be dispensed and the broad end distributes the liquid in order to generate a liquid film.

## Description

### Field of Invention

The present invention relates to an air purifier and an air purification method for use in an enclosed environment.

### Background

Home and working environments have evolved into closed air systems which re-circulate stale air including airborne particles and microorganisms trapped within these enclosed environments. Therefore, these enclosed air environments serve as pockets for particle accumulation such as dust and pollen. Dust, pollen, pet dander, mold spores, dust mite faeces and bacteria can act as allergens, triggering allergies in sensitive people. Presently, most people spend approximately 90% of their time within enclosed environment for example, in homes, offices, cars, schools and restaurants.

Investigations on the effects of indoor air quality on health, comfort and productivity have been made which resulted in the development of concepts such as "Perceived Air Quality" and "Sick Building Syndrome". These concepts have become issues of concern to the scientific and environmental communities.

As the general population becomes more affluent, greater awareness and emphasis are placed on health and well-being. Conventionally, air purifiers are used to reduce the concentration of these airborne contaminants. This is especially useful for people who suffer from allergies and asthma as it reduces the need for frequent household cleaning.

These air purifiers are also for dispersing aromatic oil to remove unpleasant smell in the enclosed environment.

WO 2005/051519 discloses a wet purifier for air purification. The wet purifier uses water to absorb the pollutants in the air into the water when the air carrying the pollutant enters the purifier. Also disclosed is a motor driving a siphon for blending the water contained in a container which results in the water reaching a centrifugal guard board through the siphon. As the centrifugal guard board rotates at a high speed, the liquid disperses from a jag, to create a water film in the container. When air containing pollutants enters the purifier and passes through the water film, the pollutants are absorbed and trapped by the water film. However, the water film is not very effective in removing most of the airborne particles and microorganisms. Additionally, the wet air purifier is unable to ensure that air drawn thereinto is completely displaced through the water film. This substantially handicaps the air purification effectiveness of the wet air purifier.

Therefore, from the foregoing, there exists a need for an improved air purifier and an improved air purifying method. The present invention provides such apparatus and method.

### Summary of Invention

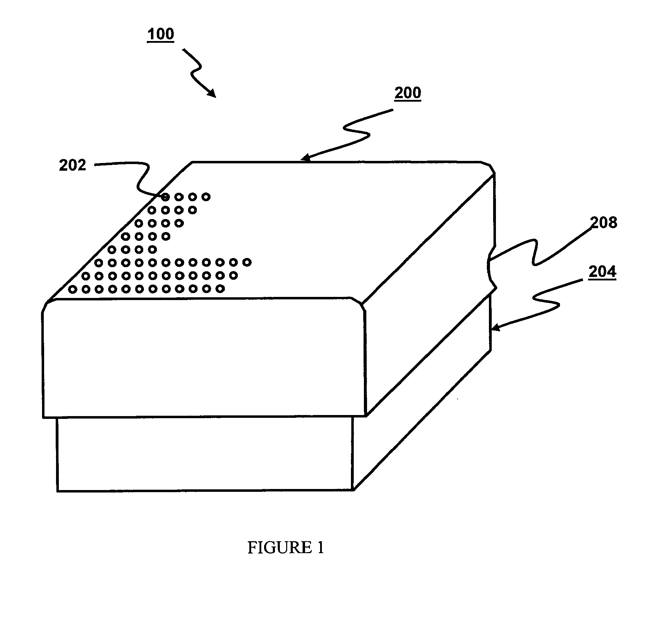

Embodiments of the invention are described hereinafter are in accordance with Figures 1 to 6 of the drawings, in which like elements are numbered with like reference numerals.

In accordance with a first aspect of the invention, there is an air purifier for use in an enclosed area which comprises a processing unit coupleable to a container containing liquid. The processing unit comprises an impeller for drawing ambient air into the processing unit, an ionizer for ionizing the ambient air to obtain first processed air and a funnel shaped for drawing liquid from the container and for substantially centrifugally expelling the liquid adjacent to the impeller for forming a liquid curtain. The impeller is for directing the first processed air through the liquid curtain to obtain second processed air. The second processed air contains liquid molecules absorbed from the liquid curtain. The second processed air is being ionized. The second processed air is subsequently exhausted into the atmosphere. The ionized second processed air traps pollutants from the atmosphere contacting. The trapped pollutants are substantially attached to the liquid molecules in the second processed air.

In accordance with a second aspect of the invention, there is a method for purifying the air which comprises the initial steps of providing liquid in a container and drawing liquid from the container through a funnel. The method also comprises the subsequent step of creating a liquid curtain with the liquid drawn through the funnel and drawing ambient air into an ionizing chamber. The liquid curtain created by centrifugally expelling the liquid from the funnel. The method further comprises the subsequent step of ionizing the ambient air to obtain first processed air. The positive ions of the ambient air are removed in the ionizing chamber which results in the air being charged with negative ions. Furthermore, the method comprises the step of displacing the first processed air through the liquid curtain to obtain second processed air. The second processed air contains liquid molecules which are absorbed from the liquid curtain. The second processed air is ionized. Finally, the method comprises the step of exhausting the second processed air into the atmosphere. The second ionized processed air traps pollutants from the atmosphere. The trapped pollutants are attached to the liquid molecules in the second processed air.

### Brief Description of the Drawings

Embodiments of the invention are described hereinafter with reference to the following drawings, in which:
FIG. 1 is a perspective view of an air purifier according to an embodiment of the invention;
FIG. 2 is a schematic drawing for an ionizer constituting the air purifier of FIG. 1;
FIG. 3 is a partial front sectional view of the air purifier with cover in FIG. 1;
FIG. 4 is a partial front sectional view of a processing unit of the air purifier of FIG. 1;
FIG. 5 is a top view of the processing unit of the air purifier in FIG. 1; and
FIG. 6 is a flowchart illustrating an air purification method used by the air purifier in FIG. 1.

### Brief Description of the Drawings

Embodiments of the invention are described hereinafter with reference to the following drawings, in which:

As shown in FIG. 1 to 6, an embodiment of the invention, an air purifier 100 for use in an enclosed environment is illustrated. The air purifier 100 comprises a cover 200 with air holes 202 and a container 204 with an opening 206. When the cover 200 is coupled to the container 204, an air inlet 208 is formed therebetween for intake of ambient air. The cover 200 further comprises a processing unit 300 for receiving ambient air from the enclosed environment for processing thereby. Processing of ambient air by the processing unit 300 preferably includes ionizing, purifying and aromatizing processes.

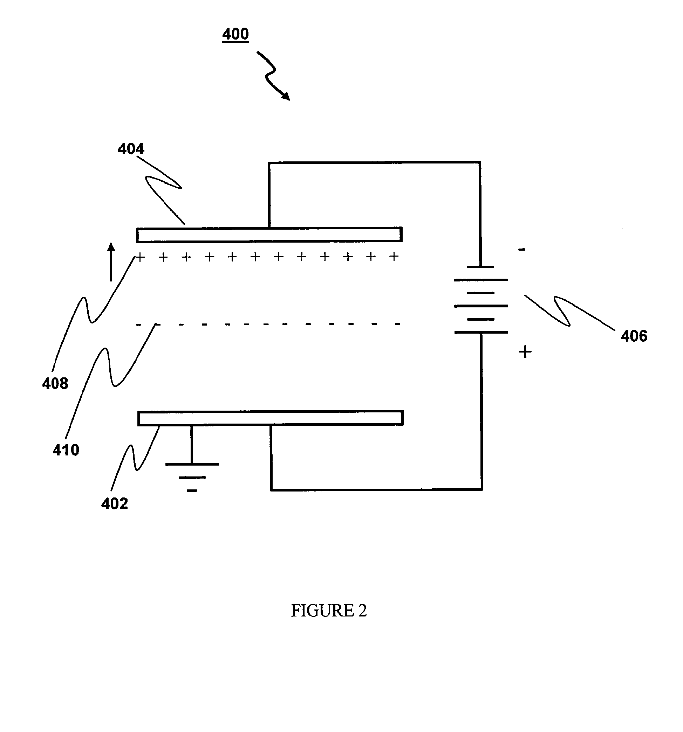

The processing unit 300 comprises an ionizing chamber 302. The ionizing chamber 302 comprises an ionizer 400 having two conducting electrodes disposed therein as shown in FIG. 2. The electrodes extending from the ionizer 400 comprises of an anode 402 and a cathode 404. When power 406 is applied thereto, an electric field is maintained between the anode 402 and the cathode 404. By grounding the anode 402, positive ions 408 are attracted to the cathode, leaving negative ions 410 when brought into contact with air. The negative ions 410 in the charged air cause the dust particles in the ambient air to agglomerate.

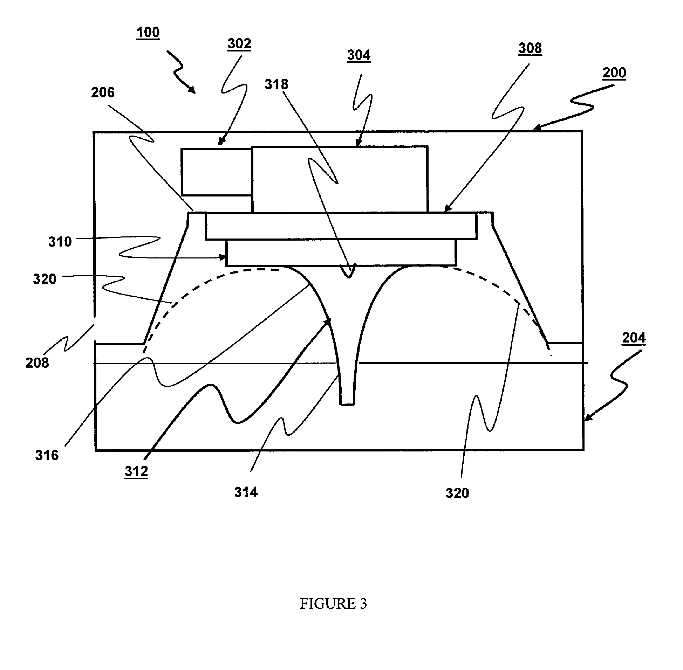

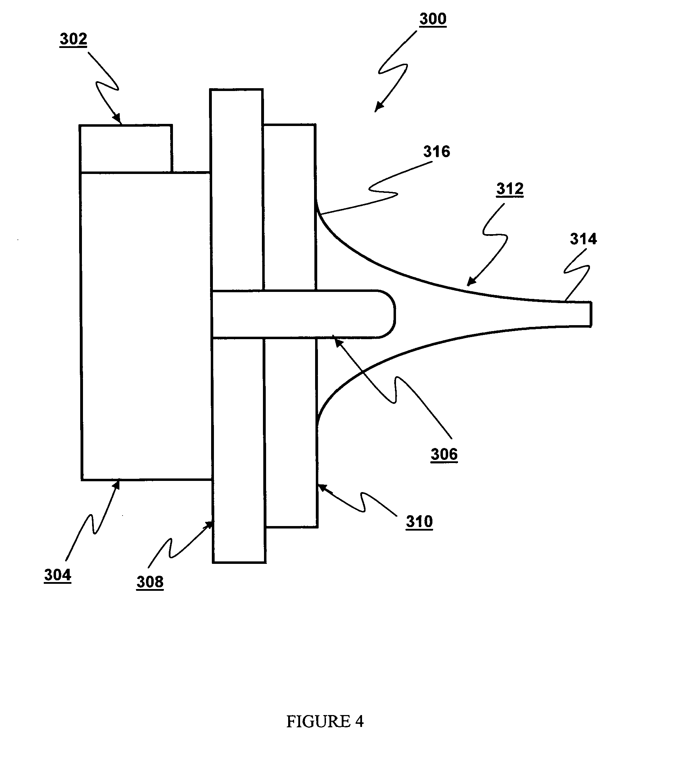

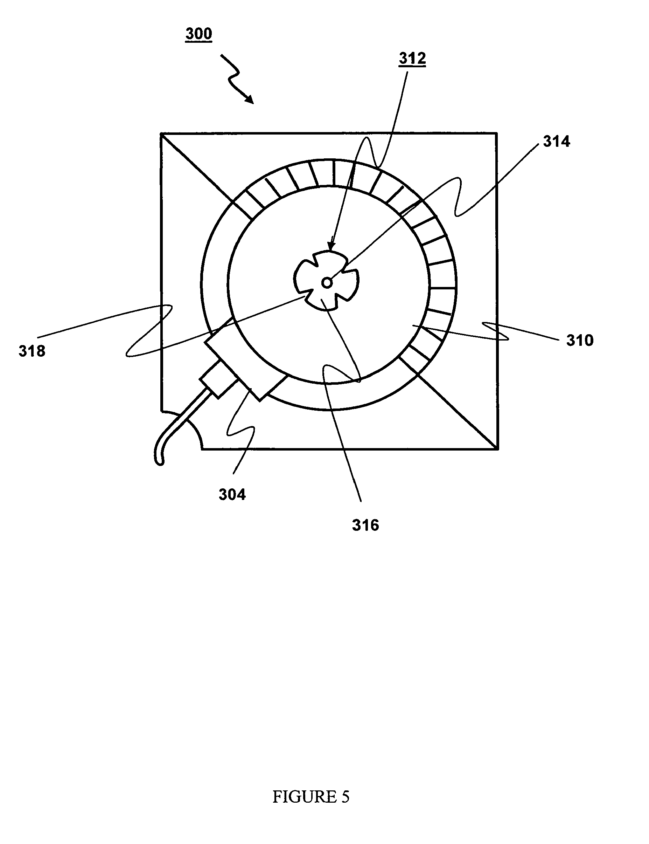

FIG. 3 illustrates the container 204 with an opening 206 for containing liquid. The liquid is preferably a water-oil mixture comprising water and aromatic oil. The water-oil mixture preferably has a specific ratio of water to aromatic oil for air purification. The processing unit 300 further comprises a motor 304 with a rotor shaft 306, an impeller 308 and a centrifugal guard board 310 coupled thereto. The processing unit 300 also comprises a funnel 312. The funnel 312 has a narrow stem 314 extending towards a conical mouth 316. Grooves 318 are formed along a portion of the funnel 312 adjacent to the conical mouth 316. The conical mouth 316 is disposed adjacent to the centrifugal guard board 310 and configured with the narrow stem 314 protruding from the processing unit 300. FIGs.4 and 5 illustrates the configuration between the motor 304, impeller 308, centrifugal guard board 310 and the funnel 312 by the rotor shaft 306. The motor 304 is for rotationally displacing the rotor shaft 306 as well as the impeller 308, centrifugal guard board 310 and the funnel 312.

When the funnel 312 is rotationally displaced, liquid is drawn from the container 204 through the funnel 312. As the centrifugal guard board 310 is also rotationally displaced, the liquid displaces out from the grooves 318 of the funnel 312, forming a liquid curtain 320. The liquid curtain 320 extends from the conical mouth 316 of the funnel 312 towards portion of the container 204 adjacent to the opening 206 of the container 204. When ambient air is drawn through the air inlet 208 and into the ionizing chamber 302, the ambient air is charged with negative ions 410 by the ionizer 400 to obtain charged air therefrom. The charged air from the ionizing chamber 302 is drawn towards the container 204 and substantially passes through the liquid curtain 320. When the charged air passes through the liquid curtain 320, pollutants in the charged air are absorbed into the liquid curtain 320 which results in processed air being obtained. The processed air is discharged back into the enclosed environment.

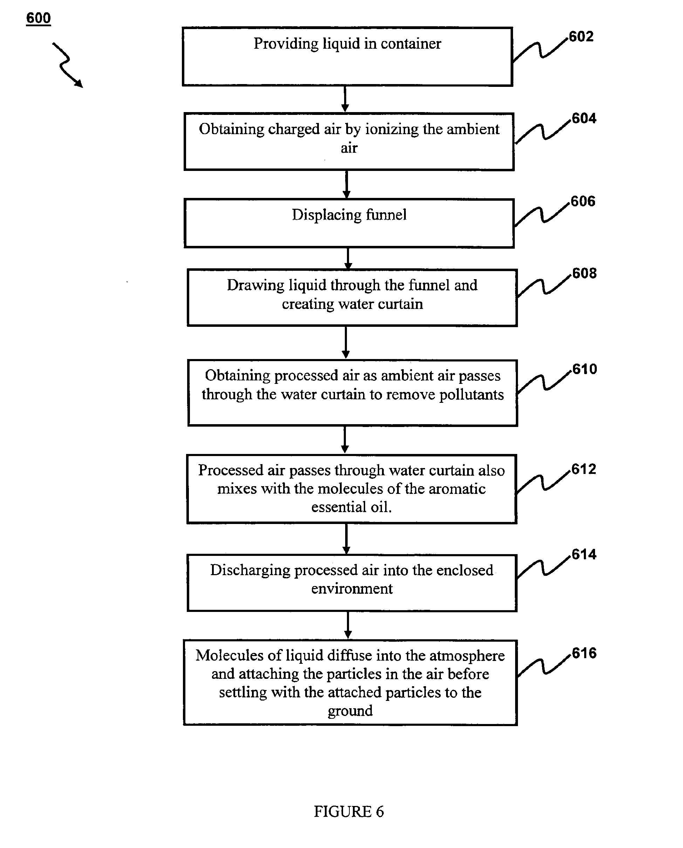

The flowchart in FIG. 6 illustrates an air purification method using the air purifier 100. Liquid is provided in the container 204 in step 602 of the air purification method 600. The ratio of water to the aromatic oil in the water-oil mixture constituting the liquid contained in the container 204 is preferably between 1:100 to 1: 150. Once the liquid is filled to a predefined level, the processing unit 300 is attached to the container 204 with a narrow stem 314 protruding and partially submerged into the liquid in the container 204.

In step 604 of the air purification method, the air purifier 100 receives ambient air containing pollutants into the processing unit 300 for ionizing. The ambient air enters into the ionizing chamber 302. The positive ions 408 in the ambient air are removed when attracted towards the cathode 404 in the ionizing chamber 302 to thereby obtained the charged air from the ambient air. Only the negative ions 410 remain in the charged air.

In step 606, the motor 304 rotationally displaces the rotor shaft 306 as well as the funnel 312. The rotational displacement of the funnel 312 results in an unidirectional rotation of the liquid. Due to the rotation of the liquid, the liquid is drawn from the container 204 through the funnel 312 towards the centrifugal guard board 310. As the centrifugal guard board 310 is also rotationally displaced, the liquid displaces out from the grooves 318 of the funnel 312, forming a liquid curtain 320 in the container 204 in step 608. The liquid curtain 320 extends from the conical mouth 316 of the funnel 312 towards portions of the container 204 adjacent to the opening thereof.

In step 610, the charged air enters into the container 204 and passes through the liquid curtain 320. The pollutants are absorbed by the liquid curtain 320 which results in removal of pollutants from the charged air. At the same instance, molecules of the liquid are also mixed with the charged air passing through the liquid curtain 320 which results in the processed air as shown in step 612. The processed air also carries the molecules of the liquid. The processed air is then discharged into the enclosed environment through the air holes 202 on the processing unit 300 in step 614.

The molecules of the liquid are subsequently diffused into the enclosed environment after the processed air is discharged thereinto. The processed air, after being discharged from the processing unit 300 and coming into contact with pollutants in the atmosphere and agglomerates the pollutants due to the negative charges 410 of the processed air. The processed air is charged with negative ions and has combined with the molecules of the liquid. When the processed air comes into contact with the pollutant in the atmosphere, the negative ions agglomerates with the pollutants in the ambient air. Due to the density of the molecules carrying the agglomerated pollutants, the molecules settle to the ground with the trapped pollutants as shown in step 616. The molecules of the liquid combined with the processed air also contain molecules of the aromatic oil which is anti-bacterial. When the molecules of the liquid is diffused into the atmosphere, the molecules of the aromatic oil substantially neutralizes the bacteria in the air in contact therewith. The molecules of the liquid and of the aromatic oil carried thereby also humidify and aromatize the air in the enclosed environment.

Therefore, purification takes place not only within the air purifier but also outside of the air purifier when the processed air carrying water molecules comes into contact with pollutants in the atmosphere.

The air purifier of the present invention has a simple construction. In particular, it can be prepared from plastic materials and manufactured using techniques such as injection molding. For purpose of brevity and clarity, the description of the invention is limited hereinafter to the use of the air purifier. This however, does not preclude the use of the apparatus in other applications. The apparatus of the present invention may be manufacture in various sizes to suit any other applications.

## Claims

1. An air purifier (100) for use in an enclosed area comprising:
a processing unit (300) coupleable to a container (204) for containing liquid,
the processing unit comprising:
an impeller (308) for drawing ambient air into the processing unit; and
an ionzier (400) for ionizing the ambient air to thereby obtain first processed air; and
a funnel (312) shaped for drawing liquid from the container thereinto and for substantially centrifugally expelling the liquid therefrom adjacent the impeller for forming a liquid curtain (320), the impeller for directing the first processed air through the liquid curtain to obtain second processed air therefrom, the second processed air containing liquid molecules absorbed from the liquid curtain, the second processed air being ionized,
wherein the second processed air is subsequently exhausted into the atmosphere, the ionized second processed air trapping pollutants from the atmosphere contacting therewith, the trapped pollutants being substantially attached to the liquid molecules in the second processed air.

2. The air purifier as claimed in claim 1, wherein the ionizer has an anode (402) and a cathode (404), the anode being grounded for the cathode to remove positive ions (408) from the first processed air attracted to the cathode.

3. The air purifier as claimed in claim 1 or claim 2, wherein the liquid in the container comprises a mixture of water and aromatic oils.

4. The air purifier as claimed in claim 3, wherein the mixture of the water and aromatic oil has a water aromatic oil ratio of between 1:100 to 1: 150.

5. The air purifier as claimed in any preceding claim, wherein the funnel has a narrow stem (314) extending towards a conical mouth (316).

6. The air purifier as claimed in claim 5, wherein grooves (318) are formed on a portion of the funnel adjacent to the conical mouth.

7. The air purifier as claimed in claim 5 or claim 6, wherein the conical mouth of the funnel is disposed adjacent to a centrifugal guard board (310).

8. The air purifier as claimed in any one of claims 5 to 7, wherein the liquid curtain extends from the conical mouth of the funnel towards portions of the container adjacent to the opening (206) thereof.

9. A method for air purification comprising:
providing liquid in a container (204);
drawing the liquid from the container through a funnel (312);
creating a liquid curtain (320) with the liquid drawn through the funnel, the liquid curtain being created by centrifugally expelling the liquid from the funnel;
drawing ambient air into an ionizing chamber (302);
ionizing the ambient air to thereby obtain first processed air;
displacing the first processed air through the liquid curtain to obtain second processed air therefrom, the second processed air containing liquid molecules absorbed from the liquid curtain, the second processed air being ionized; and
exhausting the second processed air into the atmosphere, the second ionized processed air trapping pollutants from the atmosphere contacting therewith, the trapped pollutants being substantially attached to the liquid molecules in the second processed air.

10. The method as claimed in claim 9, wherein the ionizer (400) has an anode (402) and a cathode (404), the anode being grounded for the cathode to remove the positive ions (408) from the first processed air attracted to the cathode.

11. The method as claimed in claim 9 or claim 10, wherein the liquid in the container comprises a mixture of water and aromatic oils.

12. The method as claimed in claim 11, wherein the mixture of the water and aromatic oil has a water to aromatic oil ratio of between 1:100 to 1:150.

13. The method as claimed in any one of claims 9 to 12, wherein the funnel has a narrow stem (314) extending towards a conical mouth (316).

14. The method as claimed in claim 13, wherein grooves (318) are formed on a portion of the funnel adjacent to the conical mouth.

15. The method as claimed in claim 13 or claim 14, wherein the conical mouth of the funnel is disposed adjacent to a centrifugal guard board (310).

16. The method as claimed in any one of claims 13 to 15, wherein the liquid curtain extends from the conical mouth of the funnel towards portions of the container adjacent to the opening (206) thereof.
